# EUROPÄISCHE PATENTSCHRIFT

(11) **EP 3 043 838 B1**
(45) Veröffentlichungstag und Bekanntmachung des Hinweises auf die Patenterteilung: **06.06.2018**
(21) Anmeldenummer: 14784004.5
(22) Anmeldetag: 08.09.2014
(51) Int. Cl.: A61L 31/10, A61L 27/34, A61L 27/54, B05D 1/00, C09D 105/02

(54) **GEFAESSENDOPROTHESENBESCHICHTUNG**
COATING OF A VASCULAR ENDOPROSTHESIS
REVÊTEMENT D'ENDOPROTHÈSE VASCULAIRE

(30) Priorität: 10.09.2013 DE 102013014821
(43) Veröffentlichungstag der Anmeldung: 20.07.2016
(73) Patentinhaber: Aachen Scientific International PTE. LTD., 079903 Singapore (SG)
(72) Erfinder: Rübben Alexander, 98000 Monaco (MC)
(74) Vertreter: Schneiders, Josef
(86) Internationale Anmeldenummer: PCT/EP2014/069030
(87) Internationale Veröffentlichungsnummer: WO 2015/036343

(56) Entgegenhaltungen:
- WO-A1-2008/131131
- WO-A1-2009/059625
- US-A1- 2005 015 142
- US-B1- 7 163 715
- FARAH SHADY ET AL: "Crystalline coating of rapamycin onto a stent: Process development and characterization", INTERNATIONAL JOURNAL OF PHARMACEUTICS, ELSEVIER, AMSTERDAM, NL, vol. 445, no. 1, 1 February 2013 (2013-02-01), pages 20-28, XP029001629, ISSN: 0378-5173, DOI: 10.1016/J.IJPHARM.2013.01.053

## Beschreibung

Die Erfindung betrifft ein Verfahren zur Beschichtung einer Gefäßendoprothese. Darüber hinaus betrifft die Erfindung die durch das Verfahren erhältliche Gefäßendoprothese.

Die sogenannten "minimalinvasiven Verfahren" nehmen in der Medizin einen immer größeren Stellenwert ein. So werden bei der Behandlung von Gefäßverengungen (Stenosen) häufig Gefäßendoprothesen, sog. Stents, in das Gefäß eingesetzt, um das Gefäß offen zu halten. Die Gefäßendoprothese weist typischerweise eine Röhrenform auf und besteht aus einer Geflecht- oder Gitterstruktur aus Metall oder Kunststoff. Die Gefäßendoprothese kann eine komprimierte Form einnehmen, um sie durch einen Katheter an den Zielort einführen zu können. Am Zielort wird die Gefäßendoprothese dann aufgeweitet, so dass sie ihre expandierte Form einnimmt. Das Aufweiten kann mit Hilfe eines Ballons erfolgen. Bekannt sind auch selbstexpandierende Gefäßendoprothesen, die aus einem Formgedächtnismaterial bestehen und sich von selbst entfalten, sobald sie nicht mehr in ihrer komprimierten Form gehalten oder sie einer Temperaturänderung unterworfen werden. Das Verfahren der Erweiterung von verschlossenen oder verengten Blutgefäßen mit Hilfe einer Gefäßendoprothese wird auch als Stentangioplastie bezeichnet.

Als problematisch hat sich erwiesen, dass es bei Verwendung herkömmlicher Gefäßendoprothesen häufig nach einer gewissen Zeit durch Zellproliferation und Gewebeneubildung zu Restenosen kommt, d. h. das Gefäßlumen verengt sich erneut. Dem wird versucht, durch medikamentenbeschichtete Gefäßendoprothesen vorzubeugen (sog. drug eluting stents). Insbesondere kann es sich bei den Medikamenten um Proliferationshemmer wie Paclitaxel oder auch um Immunsuppressiva wie Sirolimus handeln. Die Beschichtung kann dadurch erzeugt werden, dass der Wirkstoff in einem Lösungsmittel gelöst auf die Gefäßendoprothese aufgebracht wird und man das Lösungsmittel anschließend verdunsten lässt. Der Wirkstoff setzt sich auf diese Weise auf der Gefäßendoprothese ab und wird im Anschluss an die Implantierung nach und nach freigesetzt.

Da typische Gefäßendoprothesen eine Geflecht- oder Gitterstruktur aufweisen, der Umfang der Gefäßendoprothese somit eine Vielzahl von Durchlässen aufweist, erfolgt die Benetzung der Gefäßendoprothese mit dem Lösungsmittel, in dem der Wirkstoff gelöst ist, in der Regel sowohl innen als auch außen. Dies ist jedoch insofern unerwünscht, als der Wirkstoff auf der Innenseite der Gefäßendoprothese auch die Einbettung der Gefäßendoprothese in das körpereigene Gewebe behindert. Darüber hinaus hat sich die Kristallisation des Wirkstoffs auf Gefäßendoprothesen als schwierig herausgestellt. Hintergrund ist, dass anders als etwa bei den Ballons für die Ballonangioplastie die Oberfläche der Gefäßendoprothese hydrophil und nicht hydrophob ist, weshalb sich auf der Oberfläche der Gefäßendoprothese nicht ohne Weiteres Kristallisationskeime der typischerweise hydrophoben Wirkstoffe bilden.

Es stellte sich daher die Aufgabe, ein Verfahren zur Beschichtung von Gefäßendoprothesen zur Verfügung zu stellen, das diese Probleme aus dem Stand der Technik überwindet, so dass insbesondere der Innenraum der Gefäßendoprothese weitgehend wirkstofffrei bleibt und der Wirkstoff auf der äußeren Oberfläche gut kristallisiert.

Diese Aufgabe wird erfindungsgemäß gelöst durch ein Verfahren zur Beschichtung einer Gefäßendoprothese mit folgenden Schritten
a) zumindest teilweise Benetzung der Außenseite der Gefäßendoprothese mit einer ersten Lösung eines Wirkstoffs
b) Versetzen der Gefäßendoprothese in eine Rotationsbewegung um die Längsachse der Gefäßendoprothese
c) Ausübung einer radial wirkenden mechanischen Kraft auf die Außenseite der Gefäßendoprothese.

Im ersten, herkömmlichen Schritta) wird die Gefäßendoprothese mit einer Lösung des Wirkstoffs benetzt. Das Benetzen kann insbesondere durch Eintauchen in die Lösung oder Besprühen mit der Lösung erfolgen. Die Gefäßendoprothese wird im Schritt b) in eine schnelle Rotationsbewegung um ihre Längsachse versetzt. Die auftretende Zentrifugalkraft bewirkt, dass das Lösungsmittel, in dem derWirkstoff gelöst ist, nach außen geschleudert wird und die Innenfläche der Gefäßendoprothese praktisch wirkstofffrei bleibt. Die Entfernung überschüssigen Lösungsmittels und Wirkstoffs durch Rotation ist aus der WO 2009/059625 A1 bekannt. Die Gefäßendoprothese kann sich bereits während des Schritts a), d.h. bei der Benetzung mit der Wirkstofflösung, in Rotation befinden. Dies gilt insbesondere, wenn die Benetzung durch Eintauchen in die entsprechende Lösung erfolgt. Alternativ kann die Gefäßendoprothese auch erstnach erfolgtem Schritt a) in Rotation versetzt werden.

Im Schritt c), derzeitlich nach den Schritten a) und b) erfolgt, wird durch Ausübung einer radial wirkenden mechanischen Kraft auf die Außenseite der Gefäßendoprothese anschließend dafür gesorgt, dass sich Kristallisationskeime für den Wirkstoff bilden, so dass der (hydrophobe) Wirkstoff auf der hydrophilen Oberfläche der Gefäßendoprothese gutkristallisiert. Für die radial wirkende Kraft ist von Bedeutung, dass sie gleichmäßig über den gesamten Umfang der Gefäßendoprothese in den Bereichen einwirkt, in denen eine Benetzung stattgefunden hat. Unter radial wird eine Kraft verstanden, die von außen über den Umfang einwirkt, im Gegensatz zu einer axial wirkenden Kraft auf die Längsenden der Gefäßendoprothese.

Insbesondere kann eine radial einwirkende mechanische Kraftdadurch ausgeübt werden, dass die Gefäßendoprothese über eine Oberfläche gerollt wird. Dabei kann es sich um eine Elastomeroberfläche, beispielsweise eine Gummioberfläche handeln. Der auf die Gefäßendoprothese ausgeübte Druck sollte konstant bleiben, so dass die Kraftbeaufschlagung der Gefäßendoprothese über den gesamten Umfang gleichmäßig ist. Beim Rollen der Gefäßendoprothese über die Oberfläche werden Kristallisationskeime erzeugt, die für die Ausbildung einer kristallisierten Wirkstoffbeschichtung von

Bedeutung sind. Um beim Rollen über eine Oberfläche keine Deformierung der Gefäßendoprothese zu verursachen, ist es sinnvoll, den Innenraum der Gefäßendoprothese auszufüllen, beispielsweise mit einem in Längsrichtung eingeschobenen Stab, der vorzugsweise aus Glas oder Metall bestehen kann.

Selbstverständlich sind auch alternative Formen der Ausübung einer radial einwirkenden mechanischen Kraft denkbar. Beispielsweise kann die Gefäßendoprothese in ein entsprechendes Werkzeug eingeführt werden, das für die Ausübung einer gleichmäßigen, radial einwirkenden mechanischen Kraft geeignet ist. Die Kraft muss so bemessen sein, dass eine unerwünschte Verformung der Gefäßendoprothese nicht stattfindet.

Unabhängig von der Art der Kraftausübung, durch Rollen über eine Oberfläche oder auf andere Weise, ist typischerweise ein leichtes Andrücken ausreichend, beispielsweise mit einer Kraft von 0,5 bis 5 N, vorzugsweise 1 bis 3 N, z. B. 2 N.

Um im Innenbereich der Gefäßendoprothese nachteiligen, in einem Lösungsmittel gelösten Wirkstoff zu entfernen, wird die Gefäßendoprothese in eine Rotationsbewegung um ihre Längsachse versetzt. Die Umdrehungsgeschwindigkeit sollte mindestens 1.000 U/min, bevorzugt mind. 2.000 U/min und besonders bevorzugt mind. 5.000 U/min betragen. Als besonders vorteilhaft hat sich eine Umdrehungsgeschwindigkeit von 5.000 bis 10.000 U/min herausgestellt. Eine entsprechend hohe Rotationsgeschwindigkeit sorgt in effektiver Weise dafür, dass das den Wirkstoff enthaltende Lösungsmittel abgeschleudert wird und der innere Bereich der Gefäßendoprothese praktisch wirkstofffrei bleibt. Darüber hinaus werden auch Wirkstoffreste, die die Zwischenräume der Gefäßendoprothese ganz oder teilweise überspannen oder in diese hineinragen, entfernt. Derartige Wirkstoffreste sind unerwünscht, da es sich nicht um eine reproduzier- und quantifizierbare Wirkstoffmenge handelt. Man lässt die Gefäßendoprothese typischerweise über einen Zeitraum von 10 s bis 2 min nach der Benetzung mit der Wirkstofflösung rotieren, ein Zeitraum von 30 s hat sich als in der Regel ausreichend herausgestellt. Die Rotationsgeschwindigkeit ist erheblich höher als bei teilweise aus dem Stand der Technik bekannten Verfahren, bei denen durch eine Bewegung eine gleichmäßige Verteilung des Wirkstoffs oder ein Trocknen erreicht werden soll.

Da das Innere der Gefäßendoprothese weitgehend wirkstofffrei bleibt, wird die Gefäßendoprothese schneller mit Endothel überzogen. Im gleichen Maße verringert sich die Anregung der Blutgerinnung durch die Gefäßendoprothese. Durch Verwendung der erfindungsgemäßen Gefäßendoprothesen ist es daher möglich, schneller auf Blutgerinnungshemmer wie Acetylsalicylsäure zu verzichten. Gleichzeitig werden aber Restenosen durch die Aufbringung des Wirkstoffs auf der Außenseite der Gefäßendoprothese wirkungsvoll verhindert.

Die erste Lösung kann hinsichtlich des Wirkstoffs gesättigt sein. Als Lösungsmittel können beispielsweise Dichlormethan, Chloroform, ein Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF), Dimethylsulfoxid (DMSO), Dioxan, Dimethylformamid (DMF) oder Essigester verwendet werden. Möglich ist auch die Verwendung von Lösungsmittelgemischen. Vorzugsweise handelt es sich um eine Lösung des Wirkstoffs in Chloroform oder Dichlormethan, wobei Chloroform insofern bevorzugt ist, als es sich langsamer verflüchtigt und daher mehr Zeit verbleibt, innerhalb der das Lösungsmittel zusammen mit dem Wirkstoff durch die Rotationsbewegung nach außen geschleudert werden kann.

Eine typische Konzentration für den Wirkstoff in der ersten Lösung liegt in einem Bereich von 50 - 500 mg/ml, insbesondere 100 bis 300 mg/ml. Diese Konzentrationen haben sich bei Ausbildung einer Paclitaxel-Beschichtung bewährt. Grundsätzlich kann es sich auch um eine gesättigte Wirkstofflösung handeln.

Bei dem verwendeten Wirkstoff handelt es sich insbesondere um ein Arzneimittel bzw. Medikament, das proliferationshemmend wirkt und das gefäßverengende Überwuchern der durch die Gefäßendoprothese offen gehaltenen Stelle verhindert. Insbesondere kann der Wirkstoff ausgewählt sein aus: Tretinoin, Orphanrezeptoragonisten, Elafinderivate, Corticosteroide, Steroidhormone, Paclitaxel, Rapamycin, Sirolimus, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel bzw. Paclitaxelderivaten.

Die Beschichtung der Gefäßendoprothese mit dem Wirkstoff kann auch unter Einsatz von Lösungsvermittlern erfolgen. Als solche sind z. B. bekannt: Phosphatidylcholin, polyethoxyliertes Rizinusöl, Cardiolipin, Cholesterol sowie Gemische hieraus. Bevorzugt ist jedoch der Verzicht auf Lösungsvermittler.

Die oben genannten Verfahrensschritte, insbesondere die Schritte a) und b), können bei Bedarf auch wiederholt werden, d. h. es kann mehrfach ein Wirkstoff, der in einem Lösungsmittel gelöst ist, auf die Gefäßendoprothese aufgebracht und das Innere der Gefäßendoprothese durch Versetzen in eine Rotationsbewegung entfernt werden. Dies führt zu einer erhöhten Beladung mit Wirkstoff. Denkbar ist auch, dass nacheinander unterschiedliche Wirkstoffe aufgebracht werden.

Gemäß einer besonders bevorzugten Ausführungsform wird im Anschluss an die oben genannten Schritte a) bis c) ein Verfahrensschritt d) durchgeführt, bei dem die mit der ersten Lösung des Wirkstoffs benetzten Bereiche der Außenseite der Gefäßendoprothese mit einer Wasser und/oder mindestens einen Alkohol enthaltenden Flüssigkeit benetzt werden. Dieser Schritt sollte durchgeführt werden, nachdem die Gefäßendoprothese vollständig oder weitgehend getrocknet und der Wirkstoff nach Möglichkeit auskristallisiert ist. Die so nach der Benetzung der Oberfläche mit der ersten Lösung des Wirkstoffs entstandene Wirkstoffschicht ist eher lackartig und transparent und dient als Basis für eine homogene und reproduzierbare Wirkstoffbeladung. Die Benetzung mit der Wasser und/oder mindestens einen Alkohol enthaltenden Flüssigkeit greift die Wirkstoffoberfläche partiell an und macht diese poröser. Die Beschichtung wird somit spröder, optisch weniger transparent und milchiger. Wegen der kreideartigen Konsistenz der Oberfläche wird ein höherer Wirkstoffabtrag und eine höhere Wirkstoffabgabe an umgebende Gefäßwände erreicht, als es ohne die Benetzung mit der zusätzlichen, Wasser und/oder mindestens einen Alkohol enthaltenden Flüssigkeit der Fall wäre.

Bei der Wasser und/oder mindestens einen Alkohol enthaltenden Flüssigkeit handelt es sich insbesondere um eine einen Alkohol und/oder ein Keton enthaltende wässrige Lösung. Die Konzentration des Alkohols und/oder Ketons in der wässrigen Lösung beträgt typischerweise 10 bis 70 % (v/v), bevorzugt 20 bis 40 % (v/v) und besonders bevorzugt ca. 30 % (v/v). Verwendbar sind grundsätzlich mit Wasser mischbare Alkohole und Ketone, wobei auch eine Mischung aus mehreren Alkoholen und/oder Ketonen verwendet werden kann, für die dann die oben genannten, bevorzugten Konzentrationsangaben insgesamt gelten. Bevorzugt ist die Verwendung von Ethanol, Methanol, Aceton und/oder Isopropanol. Am meisten bevorzugt ist Ethanol. Weiterhin kann die wässrige Lösung ein azeotropes Lösungsmittelgemisch umfassen, insbesondere ein Alkohol/Wasser-Gemisch, bevorzugt ein Ethanol/WasserGemisch. Möglich ist auch der Zusatz einer kleinen Menge, typischerweise ca. 0,1 % (v/v) Essigsäure, wodurch eine Stabilisierung des Wirkstoffs, insbesondere von Paclitaxel, erreicht wird. Auch das Benetzen mit der Wasser und/oder mindestens einen Alkohol enthaltenden Flüssigkeit erfolgt typischerweise durch Eintauchen oder Besprühen. Im Anschluss an und/oder während der Benetzung kann die Gefäßendoprothese wiederum in Rotation versetzt werden bzw. sich in Rotation befinden, um eine schnellere Entfernung überschüssigen Lösungsmittels und Trocknung zu erreichen. Denkbar ist auch, dass die Flüssigkeit eine zusätzliche Menge Wirkstoff enthält, um die Beladung der Gefäßendoprothese weiter zu erhöhen.

Als weiterer Schritt e) nach dem oben beschriebenen Schritt d) können die mit der ersten Lösung und der Wasser und/oder mindestens einen Alkohol enthaltenden Flüssigkeit benetzten Bereiche der Außenseite der Gefäßendoprothese mit einer weiteren Lösung benetzt werden, die ein Polysaccharid enthält. Entsprechend wird die Gefäßendoprothese außen mit einem Polysaccharid beschichtet, wobei sich die Polysaccharidbeschichtung auf der Außenseite des Wirkstoffs befindet, d. h. der Wirkstoff ist weitgehend von einer Polysaccharidschicht abgedeckt. Ggf. kann die Gefäßendoprothese im Inneren noch durch einfaches Abwischen von der Polysaccharidbeschichtung befreit werden, da das Polysaccharid hier keine Funktion ausübt. Unbedingt erforderlich ist eine Entfernung des Polysaccharids jedoch nicht, da bereits durch den oben beschriebenen Verfahrensschritt b) hinreichend dafür Sorge getragen wurde, dass das Innere der Gefäßendoprothese praktisch wirkstofffrei bleibt.

Es hat sich herausgestellt, dass die Polysaccharidbeschichtung ähnlich einem Klebstoff auf der Innenwand des behandelten Gefäßes wirkt, d. h. der Wirkstoff haftet erheblich besser auf der Gefäßwand und wird weniger leicht vom Blutstrom mitgerissen. Vermutlich quillt das Polysaccharid, das im Gegensatz zum Wirkstoff hydrophil ist, in einer wässrigen Umgebung wie Blut leicht auf, wodurch die Übertragung auf die Gefäßinnenwand verbessert wird. Entsprechend kann der Wirkstoff über einen langen Zeitraum seine Wirkung entfalten und aus der Polysaccharidbeschichtung nach und nach in das Gewebe des Gefäßes gelangen. Es konnte gezeigt werden, dass selbst nach ca. 3 Monaten noch signifikante Wirkstoffkonzentrationen nachweisbar sind. Ohne eine Polysaccharidbeschichtung hingegen ist nach 2 bis 3 Tagen kaum noch Wirkstoff im Bereich der Gefäßinnenwand vorhanden, weshalb der durch den Wirkstoff hervorgerufene Schutz vor einer Restenose bei herkömmlichen wirkstoffbeschichteten Stents bereits nach vergleichsweise kurzer Zeit nicht mehr gegeben ist.

Ein weiterer Vorteil der Beschichtung mit einem Polysaccharid ist darin zu sehen, dass der Wirkstoff besser an der Gefäßendoprothese fixiert wird. Die Wirkstoffe, wie beispielsweise Paclitaxel, die für eine Beschichtung von Gefäßendoprothesen verwendet werden und proliferationshemmend wirken, sind häufig hoch toxisch, weshalb Ärzte und medizinisches Personal vor Einatmen und Berührung geschützt werden müssen. Durch die Abdeckung des Wirkstoffs mit einer Polysaccharidschicht wird erreicht, dass die Gefäßendoprothese problemlos gehandhabt werden kann, ohne dass sich der Wirkstoff ablöst. Das Risiko, dass Benutzer den Wirkstoff inhalieren oder über die Haut aufnehmen, wird minimiert.

Dadurch, dass zunächst der Wirkstoff und anschließend das Polysaccharid als Polymer jeweils in gelöster Form aufgebracht werden, erreicht man eine gleichmäßige Verteilung des Polysaccharids um die Wirkstoffkristalle der bereits bestehenden Wirkstoffbeschichtung. Darüber hinaus werden Hohlräume zwischen den Wirkstoffpartikeln bzw. zwischen der Oberfläche der Gefäßendoprothese ausgefüllt; die Wirkstoffkristalle werden mit dem Polysaccharid überzogen und ummantelt.

Von besonderem Vorteil ist in diesem Zusammenhang auch, dass die so erhaltene Beschichtung der Gefäßendoprothese mechanisch stabil und flexibel ist, was insofern von Bedeutung ist, als eine Gefäßendoprothese durch einen Katheter häufig durch englumige Blutgefäße an den Bestimmungsort gebracht werden müssen. Auch das Verpacken und die Handhabung der Gefäßendoprothese wird entsprechend sicherer.

Polysaccharide stellen eine hydrophile Beschichtung dar, die in einer wässrigen Umgebung wie Blut eine gewisse Quellung bzw. Aufweichung erfährt. Dies führt dazu, dass der Wirkstoff bei der Aufweitung der Gefäßendoprothese gut auf die Innenwand des Gefäßes übertragen wird. Das erfindungsgemäße Verfahren eignet sich insbesondere für lipophile Wirkstoffbeschichtungen. Es hat sich nämlich herausgestellt, dass gerade die hydrophilen Polysaccharide gut geeignet sind, dafür zu sorgen, dass lipophile Wirkstoffe während der Aufweitung der Gefäßendoprothese effektiv auf die Innenwände der behandelten Gefäße übertragen werden und eine langanhaltende Wirkstoffkonzentration bewirken. Es wird vermutet, dass, nachdem bei dem erfindungsgemäßen Verfahren zunächst die Wasser und/oder mindestens einen Alkohol enthaltende Flüssigkeit für eine Versprödung der Wirkstoffbeschichtung gesorgt hat, die anschließend aufgebrachten Polysaccharid-Moleküle sich zwischen den Wirkstoffmolekülen ablagern und so für eine homogene Verteilung des Wirkstoffs in der Polysaccharid-Matrix sorgen. Bei durch das erfindungsgemäße Verfahren hergestellten Gefäßendoprothesen wird der Wirkstoff vorteilhafterweise vom im letzten Schritt aufgebrachten Polysaccharid bedeckt.

Das Polysaccharid liegt bevorzugt in einer alkoholischen Lösung vor. Diese kann neben einem oder mehreren Alkoholen insbesondere auch Wasser enthalten. Eine wässrig-alkoholische Lösung ist insofern von Vorteil, als sie das Polysaccharid gut löst, die bereits aufgebrachte Wirkstoffschicht jedoch nicht wieder abträgt. Darüber hinaus sorgt der organische Anteil in der Lösung für eine rasche Trocknung nach der Benetzung. Die Konzentration des Alkohols bzw. der Alkohole in der wässrig-alkoholischen Lösung beträgt typischerweise 10 bis 70 % (v/v), bevorzugt 30 bis 65 % (v/v), weiter bevorzugt 50 bis 60 % (v/v) und besonders bevorzugt ca. 55 % (v/v). Als Alkohol verwendbar sind solche Alkohole, die das Polysaccharid lösen. In der Regel sind derartige Alkohole auch mit Wasser mischbar. Bevorzugt sind Ethanol, Methanol und Isopropanol, besonders bevorzugt ist Ethanol. Im Anschluss an und/oder bereits während der Benetzung mit der ein Polysaccharid enthaltenden Lösung kann wiederum die Gefäßendprothese in Rotation versetzt werden bzw. sich in Rotation befinden, um überschüssige Lösung zu entfernen und eine schnellere Trocknung zu bewirken.

Ggf. kann auch auf den Schritt d) verzichtet werden, d. h. Verfahrensschritt e) folgt direkt nach Schritt c), weil die wäßrige-alkoholische Lösung, in der das Polysaccharid gelöst ist, ebenfalls geeignet ist, die erwünschte Versprödung der Wirkstoffoberfläche herbeizuführen. In diesem Fall dient Schritt e) einem zweifachen Zweck, zum einen wird die Wirkstoffschicht poröser, zum anderen wird die Wirkstoffschicht mit Polysaccharid abgedeckt, wobei das Polysaccharid auch in die einzelnen Hohl- und Zwischenräume eindringt, die sich in der Wirkstoffschicht ausbilden.

Die mittlere Molmasse des Polysaccharids beträgt zweckmäßigerweise 10.000 bis 100.000.000 Da. Als besonders zweckmäßig hat sich eine mittlere Molmasse zwischen 20.000 und 80.000 Da herausgestellt. Bevorzugt sind verzweigte Polysaccharide. Der Polysaccharid-Gehalt der weiteren Lösung beträgt vorzugsweise 1 bis 15 Gew.-%, weiter bevorzugt 2 bis 10 Gew.-% und besonders bevorzugt 3 bis 8 Gew.-%.

Bei dem Polysaccharid handelt es sich bevorzugt um ein verzweigtes Polysaccharid. Geeignet sind auch Gemische aus mehreren Polysacchariden und modifizierte Polysaccharide. Bevorzugt sind Dextrane, insbesondere natürliche Dextrane. Bei Dextranen handelt es sich um hochmolekulare, verzweigte Polymere, die sich aus Glucoseeinheiten zusammensetzen. Sie werden u. a. von Bakterien der Gattung *Leuconostoc* hergestellt. Verwendung finden sie als Blutplasma-Ersatzmittel oder als Träger in der Chromatographie. Dextrane wirken zudem antithrombogen.

Bei dem Dextran kann es sich insbesondere um ein natürliches Dextran handeln. Besonders bevorzugt ist Dextran 40 mit einer mittleren Molmasse von ca. 40.000 Da.

Neben Dextranen können jedoch grundsätzlich auch andere Polysaccharide Verwendung finden. Ein Beispiel für ein verwendbares modifiziertes Polysaccharid ist Hydroxyethylstärke (HES).

Sämtliche Benetzungen der Oberfläche der Gefäßendoprothese mit einer Flüssigkeit (erste Lösung, Wasser und/oder mindestens einen Alkohol enthaltende Flüssigkeit oder ein Polysaccharid enthaltende weitere Lösung) können durch Eintauchen in die Flüssigkeit erfolgen. Das Eintauchen dauert dabei in der Regel max. 1 min, typischerweise 10 bis 30 s. Alternativ zur Benetzung durch Eintauchen kann die Benetzung auch auf andere Weise erfolgen, bspw. durch Besprühen oder Auftropfen. Nach den einzelnen Benetzungsschritten lässt man die Oberfläche der Gefäßendoprothese typischerweise zunächst trocknen, bevor weitere Verfahrensschritte durchgeführt werden. Das Trocknen kann durch Einwirkenlassen eines Luft- oder Gasstroms unterstützt werden. Auch das Versetzen der Gefäßendoprothese in Rotation unterstützt die Trocknung.

Bei Bedarf kann die Oberfläche der Gefäßendoprothese vor dem Aufbringen des Wirkstoffs durch mechanische, chemische oder thermische Einwirkung vergrößert, beispielsweise aufgeraut oder angeätzt werden. Auf diese Weise erhält die Oberfläche eine gröbere Struktur, so dass die Wirkstoffbeladung erhöht werden kann. Die so erzeugten Vertiefungen in der Oberfläche können bspw. eine Tiefe und einen Durchmesser von 5 - 50 µm aufweisen.

Sämtliche Verfahrensschritte können bei Raumtemperatur durchgeführt werden.

Neben dem beschriebenen erfindungsgemäßen Verfahren betrifft die Erfindung auch eine Gefäßendoprothese, deren Außenseite zumindest teilweise eine Beschichtung mit Paclitaxel bzw. mit einem Wirkstoff und einem Polysaccharid aufweist und die durch das oben beschriebene Verfahren erhältlich ist. Die Beschichtung mit dem Wirkstoff sowie auch dem Polysaccharid kann dabei die gesamte äußere Oberfläche der Gefäßendoprothese betreffen oder lediglich Teilbereiche. Von Bedeutung ist, dass das Innere der Gefäßendoprothese so weit wie möglich wirkstofffrei bleibt, um dort Endothelwachstum zu ermöglichen, das für die Einbettung der Gefäßendoprothese in das Gefäß sorgt. Auf diese Weise wird die Gefäßendoprothese schnell in das Körpergewebe integriert, wobei gleichzeitig der auf der äußeren Oberfläche der Gefäßendoprothese vorhandene Wirkstoff Restenosen durch unkontrolliertes Zellwachstum wirkungsvoll verhindert. Bei der erfindungsgemäßen Gefäßendoprothese kann es sich z. B. um einen Stent handeln, wie er im Übrigen zum Offenhalten eines Gefäßlumens bekannt ist.

Besonders vorteilhaft ist es, wenn der Wirkstoff darüber hinaus von einem Polysaccharid, insbesondere einem Dextran bedeckt wird. Das Polysaccharid bewirkt eine gute Anhaftung des Wirkstoffs an der Gefäßinnenwand, so dass eine Mitnahme des Wirkstoffs durch den Blutstrom weitgehend verhindert wird. Entsprechend können an der Gefäßinnenwand selbst nach Monaten noch erhebliche Wirkstoffkonzentrationen nachgewiesen werden, während bei herkömmlichen wirkstoffbeschichteten Gefäßendoprothesen teilweise bereits nach wenigen Tagen kaum noch Wirkstoff an der Gefäßinnenwand vorhanden ist.

## Patentansprüche

1. Verfahren zur Beschichtung einer Gefäßendoprothese mit folgenden Schritten
a) zumindest teilweise Benetzung der Außenseite der Gefäßendoprothese mit einer ersten Lösung eines Wirkstoffs
b) Versetzen der Gefäßendoprothese in eine Rotationsbewegung um die Längsachse der Gefäßendoprothese
c) Ausübung einer radial wirkenden mechanischen Kraft auf die Außenseite der Gefäßendoprothese.

2. Verfahren nach Anspruch 1, **dadurch gekennzeichnet, dass** die radial wirkende mechanische Kraft dadurch auf die Außenseite der Gefäßendoprothese ausgeübt wird, dass die Gefäßendoprothese unter Ausübung eines Drucks über eine Oberfläche gerollt wird.

3. Verfahren nach Anspruch 2, **dadurch gekennzeichnet, dass** die Oberfläche eine Elastomeroberfläche, insbesondere eine Gummioberfläche ist.

4. Verfahren nach einem der Ansprüche 1 bis 3, **dadurch gekennzeichnet, dass** die Gefäßendoprothese in eine Rotationsbewegung um die Längsachse mit einer Umdrehungsgeschwindigkeit von mindestens 1.000 U/min, bevorzugt mindestens 2.000 U/min, besonders bevorzugt mindestens 5.000 U/min versetzt wird.

5. Verfahren nach einem der Ansprüche 1 bis 4, **dadurch gekennzeichnet, dass** die erste Lösung als Lösungsmittel Chloroform oder Dichlormethan enthält.

6. Verfahren nach einem der Ansprüche 1 bis 5, **dadurch gekennzeichnet, dass** der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Sirolimus, Tacrolimus, hydrophobe Proteine und/oder zellproliferationsverändernde Substanzen.

7. Verfahren nach einem der Ansprüche 1 bis 6, umfassend als weiteren Schritt:
d) Benetzung der mit der ersten Lösung des Wirkstoffs benetzten Bereiche der Außenseite der Gefäßendoprothese mit einer Wasser und/oder mindestens einen Alkohol enthaltenden Flüssigkeit,
wobei Schritt d) nach den Schritten a) bis c) durchgeführt wird.

8. Verfahren nach Anspruch 7, **dadurch gekennzeichnet, dass** die Wasser und/oder mindestens einen Alkohol enthaltende Flüssigkeit einen Alkohol und/oder ein Keton enthält.

9. Verfahren nach Anspruch 8, **dadurch gekennzeichnet, dass** die Konzentration des Alkohols und/oder Ketons in der Flüssigkeit 10 bis 70 % (v/v), bevorzugt 20 bis 40 % (v/v) beträgt.

10. Verfahren nach einem der Ansprüche 7 bis 9, **dadurch gekennzeichnet, dass** die Flüssigkeit Ethanol, Methanol, Aceton und/oder Isopropanol enthält.

11. Verfahren nach einem der Ansprüche 1 bis 10, umfassend als weiteren Schritt:
e) Benetzung der mit der ersten Lösung und ggf. mit der Wasser und/oder mindestens einen Alkohol enthaltenden Flüssigkeit benetzten Bereiche der Außenseite der Gefäßendoprothese mit einer weiteren Lösung, die ein Polysaccharid enthält,
wobei Schritt e) nach den Schritten a) bis c) oder a) bis d) durchgeführt wird.

12. Verfahren nach Anspruch 11, **dadurch gekennzeichnet, dass** die mittlere Molmasse des Polysaccharids 10.000 bis 100.000.000 Da, bevorzugt 20.000 bis 80.000 Da beträgt.

13. Verfahren nach Anspruch 11 oder 12, **dadurch gekennzeichnet, dass** das Polysaccharid ein Dextran ist.

14. Gefäßendoprothese, deren Außenseite zumindest teilweise eine Beschichtung mit Paclitaxel aufweist, erhältlich durch ein Verfahren nach einem der Ansprüche 6 bis 13.

15. Gefäßendoprothese, deren Außenseite zumindest teilweise eine Beschichtung mit einem Wirkstoff aufweist, erhältlich durch ein Verfahren nach einem der Ansprüche 11 bis 13, **dadurch gekennzeichnet, dass** der Wirkstoff von dem Polysaccharid bedeckt wird.

## Claims

1. A method for coating a vascular endoprostheis, said method comprising the following steps:
a) at least a partial wetting of the outside of the vascular endoprosthesis with a first solution of an active substance;
b) moving the vascular endoprosthesis into a rotational movement about the longitudinal axis of the vascular endoprosthesis;
c) applying a radially acting mechanical force on the outside of the vascular endoprosthesis.

2. The method according to claim 1, **characterized in that** the radially acting mechanical force is applied to the outside of the vascular endoprosthesis by rolling the vascular endoprosthesis over a surface by exerting a pressure.

3. The method according to claim 2, **characterized in that** the surface is an elastomer surface, in particular a rubber surface.

4. The method according to any of claims 1 to 3, **characterized in that** the vascular endoprosthesis is moved into a rotational movement about the longitudinal axis with a rotational speed of at least 1,000 rpm, preferably at least 2,000 rpm, especially preferably at least 5,000 rpm.

5. The method according to any of claims 1 to 4, **characterized in that** the first solution comprises chloroform or dichloromethane as solvent.

6. The method according to any of claims 1 to 5, **characterized in that** the used active substance is chosen from the group of: tretinoin, orphanreceptoragonists, elafin derivates, corticosteroids, steroid hormones, paclitaxel, rapamycin, sirolimus, tacrolimus, hydrophobic proteins and/or cell proliferation changing substances.

7. The method according to any of claims 1 to 6, comprising as a further step:
d) wetting the areas of the outside of the vascular endoprosthesis wetted with the first solution of the active substance with a liquid comprising water and/or at least one alcohol;
wherein step (d) is executed after steps (a) to (c).

8. The method according to claim 7, **characterized in that** the liquid comprising water and/or at least one alcohol comprises an alcohol and/or a ketone.

9. The method according to claim 8, **characterized in that** the concentration of the alcohol and/or ketone in the liquid amounts to 10 up to 70 % (v/v), preferably 20 to 40 % (v/v).

10. The method according to any of claims 7 to 9, **characterized in that** the liquid comprises ethanol, methanol, acetone, and/or isopropanol.

11. The method according to any of claims 1 to 10, comprising as a further step:
e) wetting the areas of the outside of the vascular endoprosthesis wetted with the first solution and, if any, with the liquid comprising water and/or at least one alcohol with a further solution that comprises a polysaccharide;
wherein step (e) is executed after steps (a) to (c) or (a) to (d).

12. The method according to claim 11, **characterized in that** the mean molecular mass of the polysaccharide amounts to 10,000 up to 100,000,000 Da, preferably 20,000 up to 80,000 Da.

13. The method according to claim 11 or 12, **characterized in that** the polysaccharide is a dextran.

14. A vascular endoprosthesis, the outside of which has at least partially a coating with paclitaxel, obtainable by way of a method according to any of claims 6 to 13.

15. A vascular endoprosthesis, the outside of which has at least partially a coating with an active substance, obtainable by way of a method according to any of claims 11 to 13, **characterized in that** the active substance is covered by the polysaccharide.

## Revendications

1. Procédé de revêtement d'une endoprothèse vasculaire comprenant les étapes suivantes :
a) le mouillage au moins partiel du côté extérieur de l'endoprothèse vasculaire avec une première solution d'un agent actif,
b) le déplacement de l'endoprothèse vasculaire par un mouvement de rotation autour de l'axe longitudinal de l'endoprothèse vasculaire,
c) l'application d'une force mécanique à effet radial sur le côté extérieur de l'endoprothèse vasculaire.

2. Procédé selon la revendication 1, **caractérisé en ce que** la force mécanique à effet radial est exercée sur le côté extérieur de l'endoprothèse vasculaire de telle sorte que l'endoprothèse vasculaire roule sur une surface en exerçant une pression.

3. Procédé selon la revendication 2, **caractérisé en ce que** la surface est une surface élastomère, notamment une surface en caoutchouc.

4. Procédé selon l'une quelconque des revendications 1 à 3, **caractérisé en ce que** l'endoprothèse vasculaire est déplacée par un mouvement de rotation autour de l'axe longitudinal avec une vitesse de rotation d'au moins 1 000 tours/minute, de préférence d'au moins 2 000 tours/minute, de manière particulièrement préférée d'au moins 5 000 tours/minute.

5. Procédé selon l'une quelconque des revendications 1 à 4, **caractérisé en ce que** la première solution contient du chloroforme ou du dichlorométhane en tant que solvant.

6. Procédé selon l'une quelconque des revendications 1 à 5, **caractérisé en ce que** l'agent actif utilisé est choisi dans le groupe constitué par : la trétinoïne, les agonistes des récepteurs orphelins, les dérivés d'élafine, les corticostéroïdes, les hormones stéroïdiennes, le paclitaxel, la rapamycine, le sirolimus, le tacrolimus, les protéines hydrophobes et/ou les substances modifiant la prolifération cellulaire.

7. Procédé selon l'une quelconque des revendications 1 à 6, comprenant en tant qu'étape supplémentaire :
d) le mouillage des zones mouillées avec la première solution de l'agent actif du côté extérieur de l'endoprothèse vasculaire avec un liquide contenant de l'eau et/ou au moins un alcool,
l'étape d) étant réalisée après les étapes a) à c).

8. Procédé selon la revendication 7, **caractérisé en ce que** le liquide contenant de l'eau et/ou au moins un alcool contient un alcool et/ou une cétone.

9. Procédé selon la revendication 8, **caractérisé en ce que** la concentration de l'alcool et/ou de la cétone dans le liquide est de 10 à 70 % (v/v), de préférence de 20 à 40 % (v/v).

10. Procédé selon l'une quelconque des revendications 7 à 9, **caractérisé en ce que** le liquide contient de l'éthanol, du méthanol, de l'acétone et/ou de l'isopropanol.

11. Procédé selon l'une quelconque des revendications 1 à 10, comprenant en tant qu'étape supplémentaire :
e) le mouillage des zones mouillées avec la première solution et éventuellement avec le liquide contenant de l'eau et/ou au moins un alcool du côté extérieur de l'endoprothèse vasculaire avec une autre solution, qui contient un polysaccharide,
l'étape e) étant réalisée après les étapes a) à c) ou a) à d).

12. Procédé selon la revendication 11, **caractérisé en ce que** la masse molaire moyenne du polysaccharide est de 10 000 à 100 000 000 Da, de préférence de 20 000 à 80 000 Da.

13. Procédé selon la revendication 11 ou 12, **caractérisé en ce que** le polysaccharide est un dextrane.

14. Endoprothèse vasculaire, dont le côté extérieur comprend au moins en partie un revêtement avec du paclitaxel, pouvant être obtenue par un procédé selon l'une quelconque des revendications 6 à 13.

15. Endoprothèse vasculaire, dont le côté extérieur comprend au moins en partie un revêtement avec un agent actif, pouvant être obtenue par un procédé selon l'une quelconque des revendications 11 à 13, **caractérisée en ce que** l'agent actif est recouvert par le polysaccharide.
